# EUROPEAN PATENT SPECIFICATION

(11) **EP 4 476 314 B1**
(45) Date of publication and mention of the grant of the patent: **11.03.2026**
(21) Application number: 23703354.3
(22) Date of filing: 31.01.2023
(51) Int. Cl.: C12M 1/00, C12M 1/32

(54) **PLATFORM FOR SCREENING STATIC AND DYNAMIC CELL CULTURE SUPPORTS**
PLATTFORM ZUM SCREENING STATISCHER UND DYNAMISCHER ZELLKULTURTRÄGER
PLATEFORME DE CRIBLAGE DE SUPPORTS DE CULTURE CELLULAIRE STATIQUES ET DYNAMIQUES

(30) Priority: 08.02.2022 IT 202200002222
(43) Date of publication of application: 18.12.2024
(73) Proprietor: UNIVERSITA' DEGLI STUDI MAGNA GRAECIA DI CATANZARO, 88100 Catanzaro (IT)
(72) Inventor: PEROZZIELLO, Gerardo, 88021 BORGIA (CZ) (IT); CANDELORO, Patrizio, 88900 CROTONE (KR) (IT); PARROTTA, Elvira, 88100 CATANZARO (CZ) (IT); GUZZI, Francesco, 88836 COTRONEI (KR) (IT); ZACCONE, Simona, 24069 TRESCORE BALNEARIO (BG) (IT); COSENTINO, Carlo, 88100 CATANZARO (CZ) (IT); MEROLA, Alessio, 88100 CATANZARO (CZ) (IT); RANDAZZINI, Luigi, 88100 CATANZARO (CZ) (IT); CUDA, Giovanni, 88100 CATANZARO (CZ) (IT)
(74) Representative: Primiceri, Maria Vittoria
(86) International application number: PCT/IB2023/050822
(87) International publication number: WO 2023/152599

(56) References cited:
- US-A- 4 301 252
- US-A1- 2005 089 993
- US-A1- 2018 291 414

## Description

The present invention relates to a platform for screening static and dynamic cell culture supports.

In particular, the present invention relates to a platform for screening static and dynamic cell culture supports comprising hardware suitable for the miniaturization of the components used, as well as for the processing and transmission of real-time data.

The technical field is wide, since the different components of the invention pertain to the medical and pharmaceutical sectors, as well as to biochemistry, tissue engineering and real-time screening.

These sectors benefit, like others, from the continuous progress made, in particular, by the miniaturization of electronic and mechanical components, in which MEMS devices, acronym for Micro Electro-Mechanical Systems, allow to significantly reduce the size of peculiar units such as incubators for cell cultures.

The same miniaturization has led to the development of the so-called LOC, or Lab On a Chip, belonging to the MEMS sector and consisting of devices that integrate multiple functions, usually performed in a laboratory, in a single microchip whose dimensions range from a few square millimeters to a few square centimeters, designed to treat volumes of infinitesimal fluids.

The control of the mechanics of very small volumes of fluids is the basis of microfluidics, which makes use of miniaturized components such as micro-pumps, micro-valves and flow sensors, which can be integrated into LOC devices or housed in cell culture incubators.

In detail, the microclimate of a cell culture is defined by chemical and mechanical parameters and refers to a microenvironment consisting of soluble molecules around the cell, related to the composition of the cell culture medium, and of an extracellular matrix related to the composition of the culture substrate. In conventional cell cultures these environmental parameters are easily controlled for a given population of cells but cannot be monitored for small groups of cells. One of the main interests of microfluidics is to be able to control the microenvironment at the cellular level in a very specific way. Such a discipline is also able to modify the parameters influencing the cellular microenvironment in a dynamic and fully automated way, through platforms containing a series of integrated micro-devices such as the aforementioned micro-pumps and micro-valves, which allow to increase the application of this technology in chemical and biological research. Therefore, the automation and real-time control of such devices emerge significantly in order to be able to reproduce in vitro a physiological environment similar to that in vivo. It is easy to understand how the automation of a microfluidic process, through specially programmed control systems, is a fundamental and very important requirement to carry out experiments efficiently, safely and as little operator-dependent as possible.

Over the years, several microfluidic platforms have been developed in order to replicate the structure and functionality of conventional cell incubators, bringing advantages in terms of time, structures and savings. Unlike traditional cell culture methods, cell cultures in microfluidic devices offer numerous important characteristics, such as a continuous supply of nutrients, the elimination of waste products, application flexibility, liquid management systems and high automation capacity. The lower fluid consumption, low volumes and cost-effectiveness of their use make these systems particularly attractive for cellular assays. Miniaturization and spatial control are two objectives targeted by cell culture systems in microfluidic devices. Several prototypes of miniaturized incubators have been studied and designed, applying to them all the notions of microfluidics. Although microfluidic platforms capable of controlling the cellular microenvironment are available on the market, they do not always include all the instruments used for control, among other sterilization systems and specific sensor groups.

A miniaturized incubator should ideally implement the features typical of a traditional incubator for cell populations, namely:
- temperature control, which must be adjustable and generally fixed at 37°C for homo sapiens cell cultures;
- humidity control, with the miniaturized incubator that must ensure a high degree of humidity, close to 100%, avoiding evaporation or at most limiting it to the means of culture. In fact, the evaporation of H₂O from the growth medium would result in a change in the concentration of the solutes, resulting in a possible toxicity for the cells. This aspect is naturally critical in open culture systems, i.e., those systems that interface liquids with the external environment, and less critical, for example, in closed microfluidic devices;
- CO₂ control, which affects the regulation of the culture pH and is mainly obtained by bicarbonate buffering, as described in the Henderson-Hasselbalch equation:

   CO₂ + H₂O 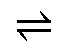

   H₂CO₃ 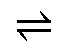 HCO₃⁻+H⁺, which shows that the equilibrium is maintained by the relationship between the concentration of CO₂ in the gas phase and the concentration of HCO₃⁻ in the cell culture medium. Acidification of the culture medium shifts the equation to the left by increasing the concentration of CO₂, while increasing the concentration of carbon dioxide in the gas phase, or in the medium due to the metabolic activity of the cells in culture, shifts the equation to the right by lowering the average pH value resulting in processing of a higher concentration of hydrogen ion H⁺ and average acidification. For an optimal buffering, the concentration of HCO₃⁻ in the culture medium should be adjusted according to the CO₂ concentration in the gas phase. It is thus evident that cell culture protocols specify different concentrations of carbon dioxide for the gas phase of the incubator, commonly 5% or 10%, in order to limit this resulting chemical mechanism, harmful to the culture itself. This aspect is crucial in static cultures and less critical in dynamic cultures, i.e., in the presence of liquid flows, but pH monitoring in all situations is very important in any case;
- pH and DO (dissolved oxygen) control, with CO₂ variation playing a key role in pH control at a neutral value of 7.4, reproducing the biochemistry of the blood flow. When the pH varies from its neutral standard value, the cells slow down growth until they lose their vitality. Continuous pH monitoring is necessary as an improper pH value can produce significant physiological changes. Measurement of dissolved oxygen, or DO, is essential in cell cultures to preserve optimal cell conditions. Low dissolved oxygen levels can affect growth rate, nutrient absorption, cell morphology and metabolite synthesis, resulting in reduced yield and lower product quality. However, high levels of dissolved oxygen result in the formation of reactive species that can oxidize media components and cause cell mutations. It is therefore essential to keep the dissolved oxygen value within a range required for an optimization of the process;
- sterile conditions, according to which the miniaturized incubator must ensure the presence of a microenvironment as sterile as possible, in order to safeguard cellular well-being and ensure the success of the culture.

Several platforms for screening cell culture supports are currently known.

A first example of a known technical solution is the subject of the US patent application US2011312764A1, which describes a LOC for microbiological analysis comprising a microfluidic device having a support substrate, an inlet for receiving a fluid, a layer made with microsystem technology, or MST, and adapted to process the fluid, with the same MST layer incorporating a section of incubation. The latter comprises at least one heater for maintaining the fluid at a predetermined incubation temperature, and a circuit made by means of CMOS technology, located between the MST layer and the support substrate, connected to the heater for the operational control of the same heater.

Furthermore, the patent application RO129999A2 describes an incubator for long-lasting cell culture experiments where it is possible to monitor the concentration of nutrients for the cells. In the incubator it is possible to introduce the cells into a biological container equipped with a series of nozzles aimed at introducing the nutrient material, the gases as well as probes for measuring parameters such as _{CO2} concentration and temperature.

The patent application US2021395656A1, instead, discloses an incubator comprising a housing space isolated from the external environment, an environmental control unit that supervises the environmental conditions inside the housing space, a power module installed in the same housing space and that supplies electric power to the control unit, and a partition wall adapted to shield the electromagnetic waves radiated from the power unit.

The patent application US2018291414A1 describes arrangements for per-well electronic sensors for microplate, microtiter, and microarray technologies are presented. In example implementations, each individual well within a conventional or specialized microplate can be fully or partially isolated with capping or other arrangement. Cap arrangements can include or provide one or more sensors of various types, including but not limited to selective gas sensors, chemical sensors, temperature sensors, pH sensors, biosensors, immunosensors, molecular-imprint sensors, optical sensors, fluorescence sensors or bio-FET sensors. Incubator interfacing and imaging are also described. The caps can also include conduits for controlled introduction, removal, and/or exchange of fluids and/or gases.

The subject of the patent application US4301252A is a miniature, biological incubator for continuous light microscopic observation of cells in cell cultures under environmentally controlled conditions for use in conjunction with petri dishes or similar types of culture dishes, in which different covers are provided to accommodate different types of microscopes and magnification, the incubator being small enough to fit on a microscope stage and to have a sufficiently small internal volume to facilitate maintenance of a controlled atmosphere having stable temperature, humidity and mixture of gases therein and to permit a rapid restoration of the controlled environment, lost during replacement of dishes or during changing of covers to suit particular microscopic observation.

The technical text describing the subject of the patent application US2005089993A1 cites a variety of microscale bioreactors, i.e., microfermentors and microscale bioreactor arrays for use in culturing cells. The microfermentors include a vessel for culturing cells and means for providing oxygen to the interior of the vessel at a concentration sufficient to support cell growth, e.g., the growth of bacterial cells. Depending on the embodiment, the microfermentor vessel may have various interior volumes less than approximately 1 ml. The microfermentors may include an aeration membrane and optionally a variety of sensing devices. The invention further provides a chamber to contain the microfermentors and microfermentor arrays and to provide environmental control.

In addition, the patent application ITNA20040016A1 relates to a microscope CO₂ incubator equipped with an internal temperature control system capable of ensuring the conditions of humidity, temperature and CO₂, or other gases, necessary for the study of biological phenomena. Temperature conditions are ensured by the internal circulation of water, or other fluid, with upstream management via temperature sensors and a PID controller. The system also includes a humidification system, by means of water-filled trays, and optical windows.

Finally, the patent application US2021032587A1 describes an incubation system for cell cultures, comprising several individual incubators and an environmental regulation system configured to control the conditions inside the chamber of each individual incubator. The environmental regulation system has a single integrated control system that manages the parameters of each incubator independently of the others. The supervised atmospheric conditions within the individual incubator include at least the oxygen level and the total gas pressure.

However, the previously described known systems suffer from some limitations, such as not providing for the housing of differentiated cellular supports, for example static and dynamic supports, bulky dimensions and the lack of electronic units responsible for a real-time control of the optimal environmental conditions of the cellular microclimate.

Another limitation of known platforms for screening cell culture supports is the reduced sterilization capacity of the cell culture environment.

A further limitation of the known platforms for screening cell culture supports is represented by the reduced interfaceability to external hardware.

The purpose of the present invention is to provide a platform for screening static and dynamic cell culture supports that allows to reproduce all the factors that characterize the cellular microclimate as such, with particular reference to the control of the parameters that influence its correct development, having, therefore, characteristics such as to overcome the limits that still affect the known technical solutions.

Another purpose of the present invention is to provide a platform for screening static and dynamic cell culture supports that allows to reproduce a contamination-free environment for the cells, in an automated mode.

A further purpose of the present invention is to provide a platform for screening static and dynamic cell culture supports employing miniaturized electronic and mechanical components and which is easily interfaceable to microscopy and spectroscopy platforms.

According to the present invention, a platform for screening static and dynamic cell culture supports is provided, as defined in claim 1.

For a better understanding of the present invention a preferred embodiment is now described, by way of non-limiting example only, with reference to the attached drawings, in which:
- figure 1 shows an overall plan view of a platform for screening static and dynamic cell culture supports, according to the invention;

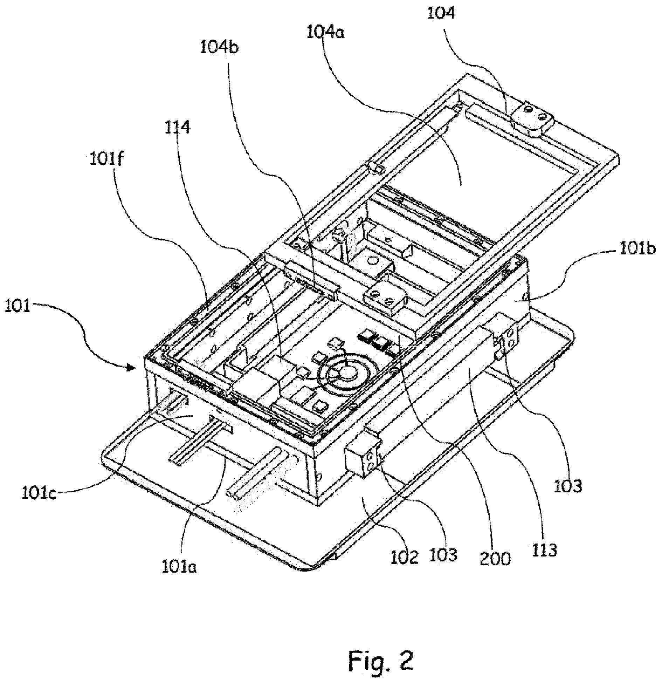

- figure 2 shows an overall perspective view of an incubator for cell cultures comprised in the platform for screening static and dynamic cell culture supports, according to the invention;

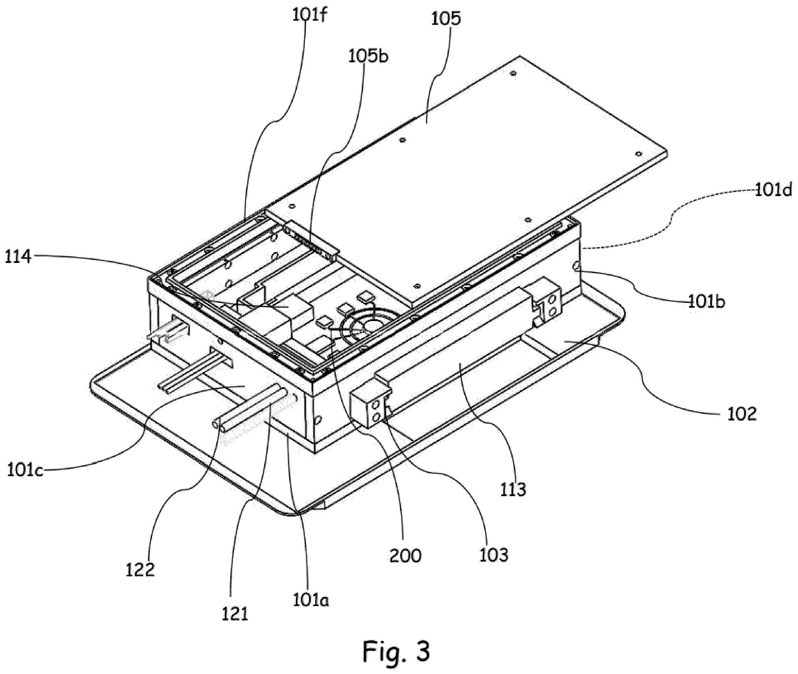

- figure 3 shows a further overall perspective view of the incubator for cell cultures, with a different cover, comprised in the platform for screening static and dynamic cell culture supports, according to the invention;
- figure 4 shows a plan view of the incubator for cell cultures comprised in the platform for screening static and dynamic cell culture supports, according to the invention;

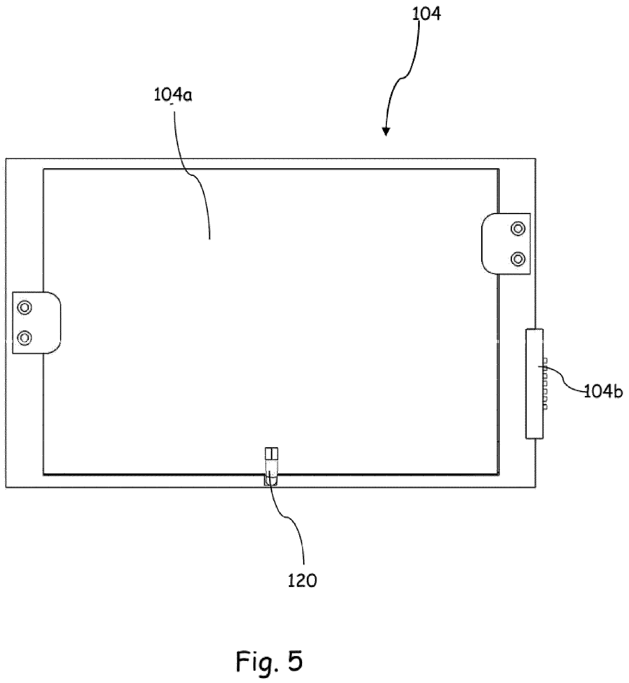

- figure 5 shows a top view of the inside of a first cover of the incubator for cell cultures comprised in the platform for screening static and dynamic cell culture supports, according to the invention;

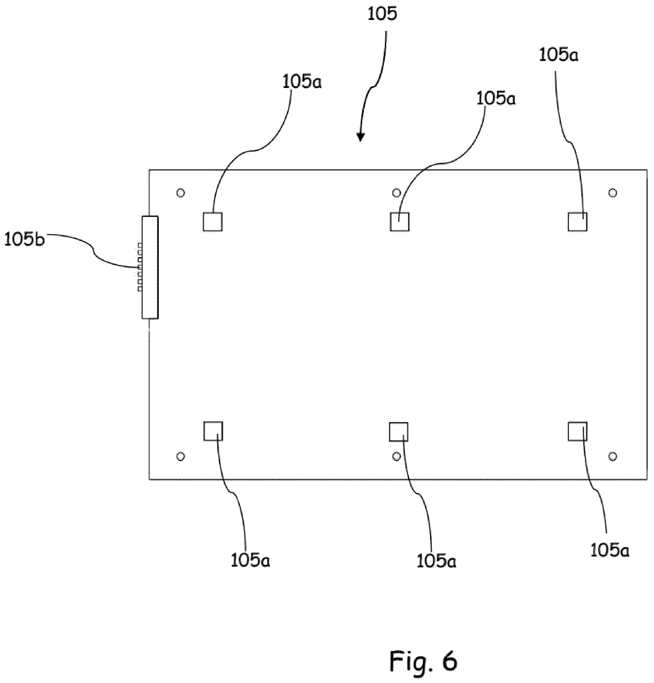

- figure 6 shows a top view of the inside of a second cover of the incubator for cell cultures comprised in the platform for screening static and dynamic cell culture supports, according to the invention;
- figure 7 shows a front sectional view of the incubator for cell cultures comprised in the platform for screening static and dynamic cell culture supports, according to the invention;

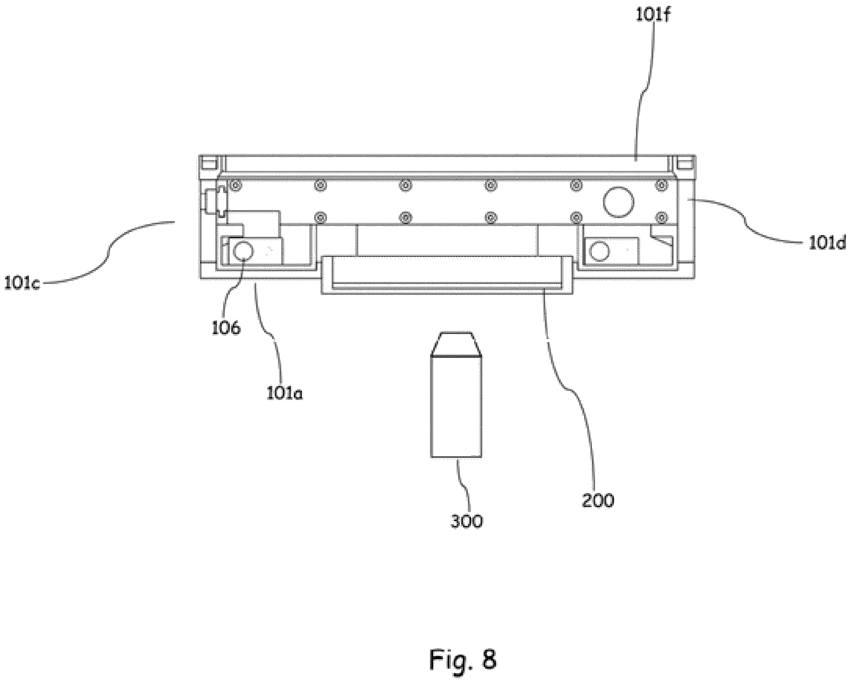

- figure 8 shows a further front sectional view of the incubator for cell cultures comprised in the platform for screening static and dynamic cell culture supports, and of part of an optical system, according to the invention;

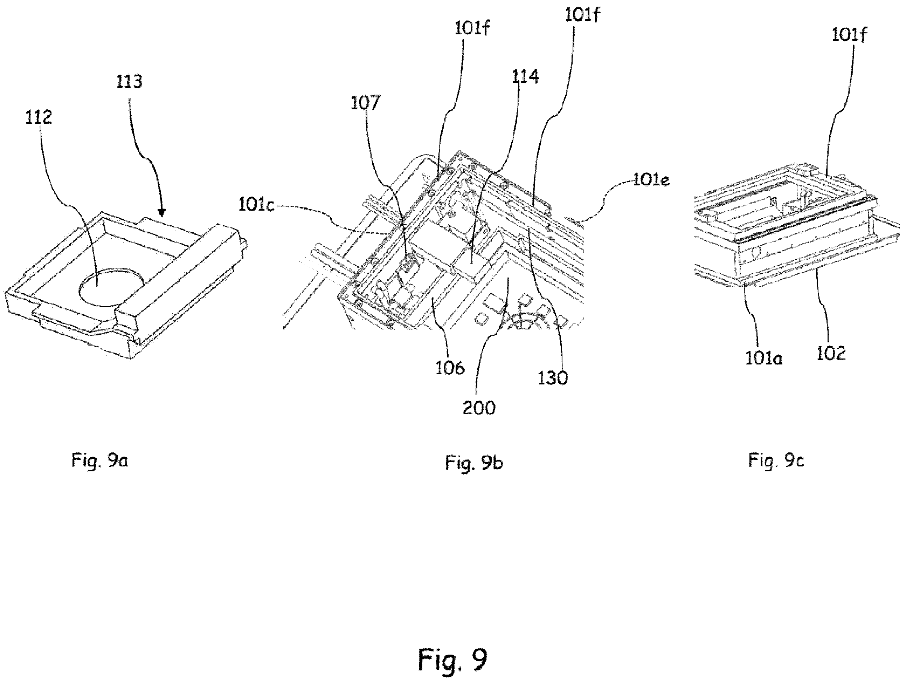

- figures 9a, 9b and 9c show perspective views relating to portions of further components of the platform for screening static and dynamic cell culture supports, according to the invention.

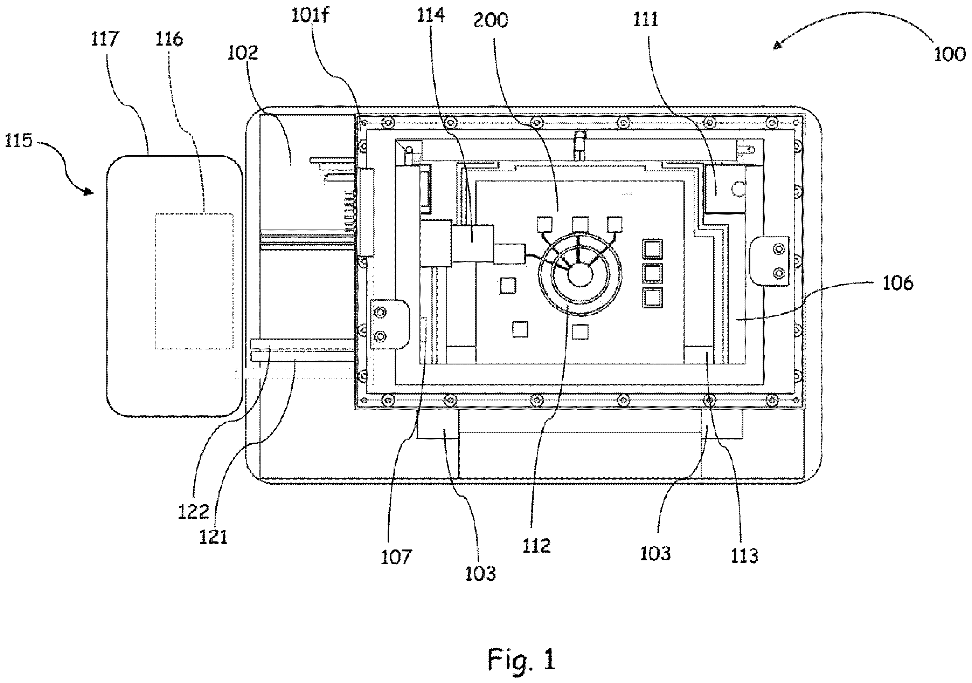

With reference to such figures and, in particular, to figure 1, a platform for screening static and dynamic cell culture supports is shown, according to the invention.

According to one aspect of the invention, the platform 100 for screening static and dynamic cell culture supports comprises:
- a miniaturized incubator 101 for cell cultures comprising a body housing a plurality of sensors, said body having a substantially parallelepiped shape and consisting of a lower panel 101a, a front panel 101b, a left side panel 101c, a right side panel 101d, a rear panel 101e and an upper panel 101f;
- a control module 115 comprising a case 117 adjacent to the body of the miniaturized incubator 101, and an electronic unit 116, housed in said case 117 and connected to the plurality of sensors;
- a base stage 102, removably fixed below the miniaturized incubator 101 in correspondence with the lower panel 101a of the body of the same miniaturized incubator 101.

According to one aspect of the invention, the miniaturized incubator 101 removably houses the static and dynamic cell culture supports.

According to one aspect of the invention, the control module 115 is positioned externally to the body of the miniaturized incubator 101 and to the base stage 102, as visible in figure 1, or fixed, as the aforementioned body of the miniaturized incubator 101, to the base stage 102 by means of an interlocking system or by means of screws.

According to one aspect of the invention, the miniaturized incubator 101 comprises a heating system, a sterilization system and a humidification system, connected to the electronic unit 116 by means of electrical cables passing through openings obtained on surfaces of the left side panel 101c, adjacent to further openings obtained on corresponding surfaces of the case 117 of the control module 115.

According to another aspect of the invention, the heating system, the sterilization system and the humidification system are configured to reproduce, inside the miniaturized incubator 101, preset and optimal conditions, typical of a microenvironment of a cell culture, regulated by a control logic running on the aforementioned electronic unit 116.

According to another aspect of the invention, the base stage 102 comprises at least one interfacing means, i.e., an adapter or a system of adapters, specifically made by means of 3D printing, removably connecting the platform 100 to at least one microscope or to a spectroscope 300.

According to another aspect of the invention, the adapter interfacing the platform 100 to a microscope or spectroscope 300 also realizes the interlocking system between the base stage 102 and the miniaturized incubator 101, and possibly also to the control module 115.

According to one aspect of the invention, the front panel 101b of the body of the miniaturized incubator 101 comprises a sliding means 113 and guides 103 for the movement of the same sliding means 113, preferably consisting of a carriage provided with an optical window 112 positioned over an external surface of the carriage 113 itself.

According to one aspect of the invention, the microscope or spectroscope 300 is, in use, removably positioned externally to the miniaturized incubator 101 in correspondence with the aforementioned optical window 112 of the carriage 113.

According to one aspect of the invention, the microscope is connected to a computer external to the platform 100, for example a PC, not shown in the figures and, in case of specific needs requiring their simultaneous presence, also to the spectroscope.

According to one aspect of the invention, the optical window 112 is preferably circular in shape, and has a diameter preferably equal to 39 mm.

According to another aspect of the invention, the static cell culture supports consist of Petri dishes, multi-well plates or slides, and the dynamic cell culture supports consist of microfluidic devices 200, or MFDs.

According to one aspect of the invention, the static and dynamic cell culture supports can alternatively be housed, according to specific needs, removably inside the miniaturized incubator 101 by means of the sliding means 113 which facilitates their entry and exit from the same miniaturized incubator 101 after being placed on the sliding means 113. The carriage 113, in other words, supports and introduces removably, inside the miniaturized incubator 101, alternatively the static and the dynamic cell culture supports.

Advantageously according to the invention, the guides 103 obtained in the front panel 101b allow the carriage 113 to reach an optimal height for focusing and viewing the cell cultures, present in the static and dynamic cell culture supports, by means of optical systems, e.g., the microscope or spectroscope 300.

According to one aspect of the invention, the upper panel 101f is hollow, and comprises further guides for the removable insertion of covering elements of the body of the miniaturized incubator 101, in particular a first covering element 104 and a second covering element 105, relative respectively to the heating system and to the sterilization system, configured to slide in the further guides of the upper panel 101f to close the body of the miniaturized incubator 101, and to perform further dedicated functions, reported hereafter.

According to one aspect of the invention, the heating system comprises the first covering element 104, which is provided with a transparent and electrically conductive heating glass 104a and which is configured to regulate the temperature of the microenvironment of the cell culture inside the miniaturized incubator 101.

According to another aspect of the invention, the sterilization system comprises the second covering element 105, which is provided with a plurality of LEDs 105a configured to generate an UV-C radiation, which allows to sterilize the microenvironment of the cell culture inside the miniaturized incubator 101.

According to another aspect of the invention, the humidification system comprises a bowl 106 able to contain water, and heating elements 111 configured to heat the same water, fixed inside the bowl 106.

According to another aspect of the invention, the miniaturized incubator 101 comprises, fixed in correspondence with a lower portion of the upper panel 101f, a film made of FEP, Fluorinated Ethylene Propylene, not shown in the figures and configured to close the miniaturized incubator 101 when the first covering element 104 or the second covering element 105 are not inserted in the upper panel 101f through the further guides of the latter.

According to another aspect of the invention, in use, with the first covering element 104 or the second covering element 105 positioned inside the further guides of the upper panel 101f, consequently re-closing the miniaturized incubator 101, the film made of FEP is located below the aforementioned first covering element 104 or second covering element 105.

Advantageously according to the invention, the film made of FEP also allows the passage of the UV-C radiation, generated by the sterilization system, when the second covering element 105 is inserted in the upper panel 101f.

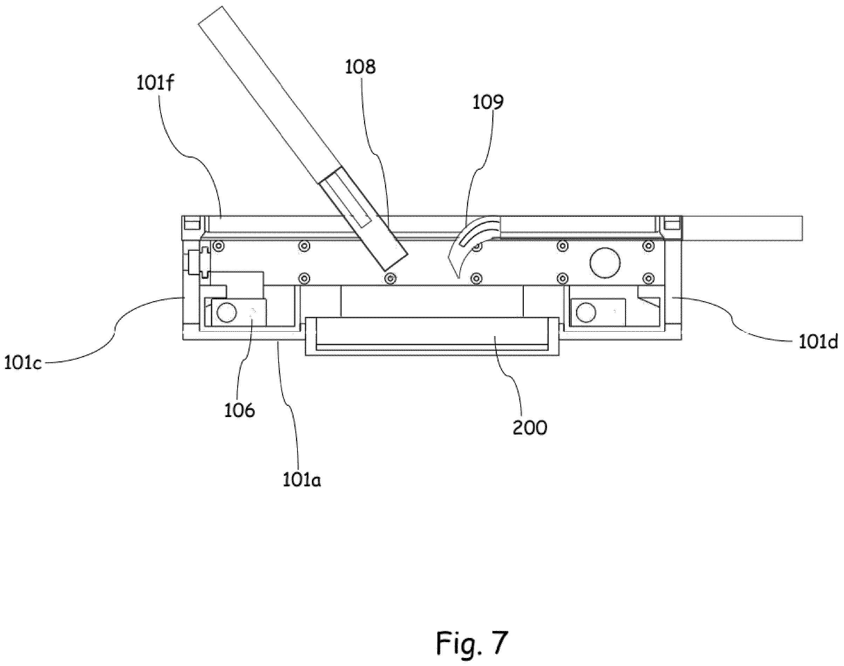

According to another aspect of the invention, the sensors housed in the body of the miniaturized incubator 101 are covered by flexible coating sheaths, while portions of such sheaths, in particular those covering the elements sensitive to the variable to be detected, are housed inside the miniaturized incubator 101. The remaining portions of such sheaths, essentially covering electrical conductors connected to the electronic unit 116 that acquires the signals, come out of the miniaturized incubator 101 by means of the openings obtained on respective surfaces of the left side panel 101c and the right side panel 101d, as visible in figure 7, and which converge in an I/O section of the same electronic unit 116 through further openings obtained on corresponding surfaces of the case 117 of the control module 115.

Advantageously according to the invention, a second flexible coating sheath is provided inside each flexible coating sheath to protect the sensitive elements of the sensors.

According to another aspect of the invention, the electronic unit 116 is configured to run a proportional integral derivative PID control logic for a real-time management of the heating system, the sterilization system and the humidification system of the miniaturized incubator 101.

According to another aspect of the invention, the plurality of sensors comprises:
- a pH sensor 108;
- a DO, Dissolved Oxygen, sensor 109;
- a temperature and humidity sensor 114, configured to detect the humidity and temperature of the microenvironment of the cell culture inside the miniaturized incubator 101;
- a level sensor 107, preferably a capacitive sensor, configured to detect the level of the water contained in the bowl 106;
- a temperature sensor 120, fixed to the heating glass 104a, able to detect the temperature of said heating glass 104a;
- a thermocouple, connected to the heating elements 111 able to heat the water contained in the bowl 106, configured to detect the temperature of said water.

According to another aspect of the invention, the platform 100 comprises a micro-pump, connected to a tank and to the electronic unit 116, positioned externally to the miniaturized incubator 101 and able to introduce water inside the bowl 106, according to an automated filling managed by means of the PID control logic running on the aforementioned electronic unit 116.

According to another aspect of the invention, the platform 100 comprises a solenoid valve, connected to the electronic unit 116, configured for the introduction through pipes 121 of a 5% CO₂ mixture into the microenvironment of the cell culture, with the aforementioned CO₂ mixture being able to regulate the pH of the incubated cell culture.

According to another aspect of the invention, the bottom panel 101a, the front panel 101b, the left side panel 101c, the right side panel 101d, the rear panel 101e, the top panel 101f and the base stage 102 are made of PMMA by micro-milling and 3D printing.

According to another aspect of the invention, the microscope or spectroscope 300 is configured for real-time observation of static and dynamic cell culture supports through the optical window 112 comprised in the carriage 113 of the front panel 101b.

According to another aspect of the invention, the platform 100 comprises a wireless data transmission unit, connected to the electronic unit 116, and at least one data cloud storage module able to save data processed by the electronic unit 116 and transmitted to said data cloud storage module by means of the wireless data transmission unit.

According to another aspect of the invention, the body of the miniaturized incubator 101 has a plan section having a length comprised between 143 mm and 223 mm, preferably equal to 183 mm, and a width comprised between 80 mm and 160 mm, preferably equal to 120 mm.

According to another aspect of the invention, the body has a height comprised between 22 mm and 62 mm, and preferably equal to 42 mm.

According to another aspect of the invention, the base stage 102 has a substantially rectangular shape having a length comprised between 205 mm and 265 mm, preferably equal to 235 mm, and a width comprised between 124 mm and 184 mm, preferably equal to 154 mm.

In detail, in terms of size, the known cell culture incubators have bulky dimensions, a feature that certainly does not make them easy to transport or connect to other instruments. In the specific case of the miniaturized incubator 101 according to the invention, the substantially parallelepiped-shaped body has small dimensions, preferably equal to 183 mm x 120 mm in plan, with a height of 42 mm, which make it effectively transportable. The miniaturized incubator 101, based on what previously described, consists of several components, which can be made of different plastic or metallic materials, and preferably, as mentioned, of PMMA sheets processed with micro-milling techniques, and with 3D printed structures in biobased "Filoalfa AlfaPRO" techno-polymer. A viable alternative to such materials is aluminum or steel, particularly for components that do not need to be transparent. Steel, in particular, is characterized by excellent thermal resistance to high temperatures, of the order of 115°C with annealing process, ease of printing without the release of any bad odor, consequent non-toxicity, excellent mechanical properties of torsion and tensile strength, resistance to hydrolysis and UV rays.

As anticipated, the various components of the miniaturized incubator 101 are made by means of micro-milling and 3D printing techniques and are assembled through screws. In particular, during micro-milling, numerical control machines performing set machining programs are used. The tips of a spindle used in the process can range in size from a few micrometers to several millimeters in diameter. The FDM 3D-printing step allows to quickly produce objects without constraints on shape, and thanks to the wide variety of materials that can be used, it is possible to select technical, mechanical and color characteristics that are better suited to design needs. In the post-processing step, after production, the components are perfected by cleaning the support using sandpaper and files to correct imperfections, an annealing process to reinforce the material and the threading of the holes.

According to one aspect of the invention, the electronic unit 116 of the control module 115, adjacent to the miniaturized incubator 101 on the base stage 102 or external to the latter, is made by means of a PCB card based on a microcontroller with ARM architecture, which, in addition to interfacing the plurality of sensors and managing their respective detections, comprises specific amplifiers and transistors for the real-time automated control, by means of the PID logic running on the same microcontroller, of the operation of the platform 100. The entire electronic section is powered by a specific 12 Vdc power supply, afterwards a series of voltage regulators provides the correct voltage to the different components, for example 5 Vdc to power the microcontroller and the display, 12 Vdc for the heating elements 111, 3.3 Vdc for the sensors and the buzzer, about 6.5 Vdc for the UV-C LEDs 105a. A pair of fuses is also provided to protect against any overcurrents.

Advantageously according to the invention, the miniaturized incubator 101 reproduces the fundamental parameters of the cellular micro-environment substantially through the three systems previously outlined:
- the heating system, and the miniaturized incubator 101 as a whole, must ensure a temperature of 37°C, optimal for homo sapiens cells. The desired temperature is obtained using the heating glass 104a, positioned in the first covering element 104 and suitably connected to the electronic unit 116 of the control module 115 by means of magnetic connectors 104b with Pogo Pin, and corresponding magnetic connectors positioned on the upper panel 101f of the body of the miniaturized incubator 101, which allow a simple contact and, thanks to the magnets, keep in position both the first covering element 104 and the second covering element 105, referable as interchangeable covers of the miniaturized incubator 101. The heating elements 111 placed in water also contribute to the heating of the microenvironment of the cell culture on static and dynamic supports;
- the humidification system helps to ensure a humidity of the microenvironment of the cell culture close to 100%, to avoid or reduce evaporation of the culture medium and prevent alteration of the concentrations of dissolved solutes, with potential toxic effects for the cells. The water is introduced into the system, by means of an automatic refill mechanism managed by the PID logic of the electronic unit 116, through the volumetric micro-pump placed outside the miniaturized incubator 101, considering the information coming from the level sensor 107 integrated in the tank 106. Inside the latter, the heating elements 111 are able to raise the temperature of the water contributing to reach the target temperature in the microenvironment, increasing the evaporation of the water to saturate the microenvironment more quickly;
- the sterilization system, before and after each use of the miniaturized incubator 101, prevents contamination of the cell cultures. The sterilization takes place by means of the ultraviolet radiation emitted by the UV-C LEDs 105a placed in the second covering element 105, as better described in the following. Even in this case, the covering element 105 with the UV-C LEDs 105a is connected to the magnetic connectors of the top panel 101f by means of second magnetic connectors 105b with Pogo Pin. The control module 115 allows to set the turning on or off of the LEDs 105a even at a preset time, and to plan their duration of activity. To preserve the sterility of the cell culture microenvironment during the replacement of the UV-C covering element 105 with the covering element 104 integrating the heating glass 104a, and vice versa, the film made of FEP is provided to close the miniaturized incubator 101. This component, in addition to avoiding contamination when one of the covering elements is not in place, ensures the passage of UV-C radiation for the sterilization of the microenvironment, being transparent to the wavelengths of this ultraviolet bandwidth.

Advantageously according to the invention, the optical window 112 obtained on an external surface of the front panel 101b allows the visualization of the cell cultures present in the static and dynamic cell culture supports, that is, respectively, the Petri dishes, multi-well plates or slides, and the microfluidic devices 200, or MFDs.

Advantageously according to the invention, the possibility of interfacing the platform 100 with an optical microscope, a fluorescence microscope or a spectroscope, for example a Raman spectroscope, allows to obtain information also from a biochemical, and not only phenotypic, point of view, of phenomena characterizing the microenvironment of cell cultures.

According to one aspect of the invention, the left side panel 101c, in addition to the openings obtained also on the right side panel 101d that allow the passage of the sheaths coating the sensors between the outside and inside of the miniaturized incubator 101, in the direction of the electronic unit 116, houses a pocket capable of housing the electrical cables coming from the inside of the miniaturized incubator 101, in particular from the three environmental heating, sterilization and humidification systems, and guiding them towards the outside. The rear panel 101e also houses a further pocket capable of performing the same function.

According to another aspect of the invention, the platform 100 also comprises an electrical cable covering plate 130, used to lock and isolate the electrical cables inside the aforementioned pockets made on the rear panel 101e and on the left side panel 101c. The insulation is increased thanks to the use of specific gaskets and cables suitable for working in high humidity and/or immersion conditions.

According to one aspect of the invention, the bowl 106 of the humidification system, which acts as a moisture source for the environment, is made through 3D printing and comprises structures supporting other components/sensors of the miniaturized incubator 101. In particular, such components comprise fixing systems for the heating elements 111, further pipes 122 for filling and suctioning the water, a load-bearing structure for the temperature and humidity sensor 114 and for the level sensor 107. Any issue of leakage of the bowl 106, due to the porosity of the material used and to the manufacturing technique, FDM, can be solved by a hydrophobic coating, for example based on PMMA or COC dissolved in solvent. After their deposition, the evaporation of the solvent promotes the solidification of the polymeric material, creating a polymeric film that prevents water molecules from penetrating inside it. In addition, a hydrophobic treatment can help to limit the capillary forces that would form between the water contained within the bowl 106 and the material in which it is made, preventing the water from leaking when it is close to the upper edge of the same bowl 106.

Advantageously, according to the invention, the water heating elements 111 also promote evaporation.

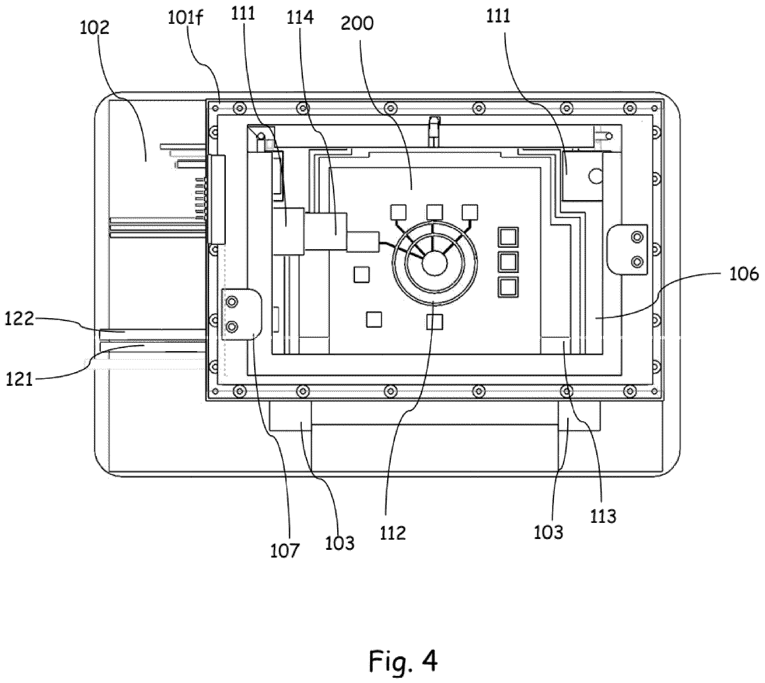

According to one aspect of the invention, the temperature and humidity sensor 114 projects towards the central zone of the miniaturized incubator 101, as visible in figure 4, where the cells are placed in culture and where it is necessary to have more accurate information on the parameters to be monitored.

According to one aspect of the invention, the film made of FEP is fixed to the lower portion of the upper panel 101f by means of specific structures, and screws, or by means of double-sided tape.

According to one aspect of the invention, the UV-C LEDs 105a of the sterilization system are mounted in such a way as to maximize their area of action and fully sterilize the interior of the miniaturized incubator 101. In particular, the UV-C LEDs 105a are not soldered on a PCB board, which is mounted in the second covering element 105, or UV cover 105, but integrated by SMD technology on plates, with the function of adapters, insertable in the second UV cover 105 itself with an interlocking mechanism that allows, in the event of damage, an easy replacement. On the PCB integrated in the UV cover 105 are also placed contact connectors for CR1216 or CR1220 batteries, which also represent the insertion points of the plates. The latter are provided with two pins with 2.54 mm pitch, on the opposite side to the contact with the aforementioned connectors, useful for carrying out any tests that do not involve the use of the miniaturized incubator 101.

Advantageously according to the invention, the heating glass 104a, being transparent, allows an optimal visualization of the interior of the miniaturized incubator 101.

According to one aspect of the invention, the various electrical components placed on the first covering element 104 and on the second covering element 105, or interchangeable covers, are actuated and powered by means of their respective magnetic connectors 104b, 105b placed thereon, alternately brought into contact with the magnetic connectors of the top panel 101f, as previously described. In detail, the magnetic connectors with Pogo Pin ensure a simple contact that, thanks to the magnets, keeps in place the interchangeable covers 104 and 105 to close the body of the miniaturized incubator 101.

According to one aspect of the invention, the pH sensor 108 and the DO sensor 109, which detects dissolved oxygen, can be integrated from above, making heating glasses 104a with customized holes, or they can be integrated laterally, depending on the needs and the available dimensions. The sensors are inserted by means of suitable flexible sheaths so as to easily reach the positions of interest inside the miniaturized incubator 101. Each sensor, as mentioned, slides inside a further guide, made inside the first outermost one, in such a way as to distance and protect the sensitive and fragile part of the sensor from the walls of the outer guide. As an alternative to fiber sensors, a material sensitive to pH and DO, whose variations in optical properties can be detected by the microscope and/or spectroscope interfaced to the platform 100, can be introduced into the miniaturized incubator 101.

According to one aspect of the invention, the platform 100 comprises a buzzer, connected to the electronic unit 116, which provides a sound feedback to laboratory operators, with the purpose of drawing attention in case specific events occur.

According to one aspect of the invention, the platform 100 comprises a touchscreen display, not shown in the figures, positioned on an upper portion of the container 117 of the control module 115 adjacent to the body of the miniaturized incubator 101, able to provide information on the status of the miniaturized incubator 101, quickly manage the parameters, display the water level in the bowl 106 and initiate specific functions, for example the sterilization process via the UV-C LEDs 105a.

According to one aspect of the invention, the tank of the humidification system may consist of an Eppendorf tube or other containers filled with water, reagents or samples to be introduced into the miniaturized incubator 101. As for the reagents, when the micro-pump is operated the air is pumped into the tank, increasing the pressure therein with consequent introduction of the aforementioned reagents into the miniaturized incubator 101.

According to one aspect of the invention, as anticipated, the pocket of the left side panel 101c, specific for the electrical cables, conveys the same cables in three blocks, i.e., three cables coming from the water level sensor 107 reach an amplifier board for capacitive tactile sensors, other cables reach a twelve-pole circular connector for the sensors, and the remaining cables arrive at a DB9 connector positioned on a PCB equipped with terminals, for power supply. The choice of the positioning of the amplifier board for capacitive tactile sensors, as an alternative to the positioning on the main electronic unit 116 of the control module 115, has the advantage of a reduction of connection cables between the miniaturized incubator 101 and the electronic unit 116 itself, the reduction of possible noise causes from the capacitive sensor thanks to the optimization of the length of the cables connected to the level sensor 107, as well as the possibility of inserting buttons above the miniaturized incubator 101 on a second time.

Advantageously according to the invention, the electronic unit 116 alerts laboratory operators, based on the detections of the level sensor 107 if the water level in the bowl 106 exceeds or is below preset thresholds.

Advantageously according to the invention, the data saved on the cloud data storage module is remotely manageable and accessible, for example by means of a web browser.

Therefore, the platform for screening static and dynamic cell culture supports according to the invention allows to reproduce all the factors that characterize a cellular micro-environment, paying a high attention to all the parameters that influence its correct development and reproducing an environment free of contamination for the same cells.

A further advantage of the platform for screening static and dynamic cell culture supports according to the invention is that it allows to monitor and control in an automated way, by means of dedicated electronics and logic, the parameters of temperature, humidity and the concentrations of chemical species of interest present in the cell growth means.

Another advantage of the platform for screening static and dynamic cell culture supports according to the invention is that, thanks to a sliding means, microfluidic devices, Petri dishes, multi-well plates and slides can be introduced inside the miniaturized incubator without the need to remove the covering elements of the same incubator.

A further advantage of the platform for screening static and dynamic cell culture supports according to the invention is that it is easily interfaceable to different platforms for microscopy and spectroscopy.

Furthermore, the interoperability of the platform for screening static and dynamic cell culture supports according to the invention with spectroscopes means that the miniaturized incubator represents an important resource for real-time monitoring and for the characterization of cell growth, also from a biochemical and not only phenotypic point of view.

Finally, the platform for screening static and dynamic cell culture supports according to the invention is usable for monitoring, in a controlled environment, drug-screening processes, tissue-engineering, as well as cell expansion, reprogramming and differentiation.

It is finally clear that the platform for screening static and dynamic cell culture supports, described and illustrated herein, may be subject to modifications and variations without departing from the protective scope of the present invention, as defined in the appended claims.

## Claims

1. Platform (100) for screening static and dynamic cell culture supports, comprising:
- a miniaturized incubator (101) for cell cultures comprising: a body comprising a front panel (101b); a plurality of sensors housed in the body; and a heating system, a sterilization system and a humidification system configured for reproducing inside said miniaturized incubator (101) preset and optimal conditions, typical of a microenvironment of a cell culture;
said miniaturized incubator (101) removably housing static and dynamic cell culture supports;
- a control module (115) comprising: a case (117) adjacent to the body of the miniaturized incubator (101); and an electronic unit (116) housed in said case (117) and connected to the plurality of sensors, to the heating system, to the sterilization system and to the humidification system;
- a base stage (102), removably fixed below the miniaturized incubator (101), comprising at least one interfacing means consisting of an adapter or a system of adapters removably connecting said platform (100) to at least one microscope or spectroscope (300);
**characterized in that** the front panel (101b) comprises a sliding means (113) and guides (103) for the movement of the sliding means (113), said sliding means (113) consisting of a carriage, supporting and introducing in a removable way inside the miniaturized incubator (101), alternatively, said static and dynamic cell culture supports, provided with an optical window (112) positioned over an external surface of said carriage (113), in correspondence with said optical window (112) being removably positioned, externally to the miniaturized incubator (101), the at least one microscope or spectroscope (300).

2. Platform (100) according to claim 1, **characterized in that** the static cell culture supports are made up of Petri dishes, multi-well plates or slides, and **in that** the dynamic cell culture supports are made up of microfluidic devices (200).

3. Platform (100) according to claim 1, **characterized in that** the body has a substantially parallelepiped shape and consists of a lower panel (101a), a left side panel (101c), a right side panel (101d), a rear panel (101e), an upper panel (101f) and the front panel (101b).

4. Platform (100) according to claim 3, **characterized in that** the base stage (102) is removably fixed below the miniaturized incubator (101) in correspondence with the lower panel (101a).

5. Platform (100) according to claim **3, characterized in that** the upper panel (101f) is hollow and comprises further guides for a removable insertion of covering elements configured to cover the body of the miniaturized incubator (101).

6. Platform (100) according to claim **5, characterized in that** the heating system comprises a first covering element (104) of the body of the miniaturized incubator (101), able to slide into the further guides of the upper panel (101f), provided with a transparent and electrically conductive heating glass (104a), said heating glass (104a) being able to regulate the temperature of the microenvironment of the cell culture inside the miniaturized incubator (101).

7. Platform (100) according to claim 5, **characterized in that** the sterilization system comprises a second covering element (105) of the body of the miniaturized incubator (101), able to slide into the further guides of the upper panel (101f), provided with a plurality of LEDs (105a), said plurality of LEDs (105a) being configured to generate an UV-C radiation able to sterilize the microenvironment of the cell culture inside the miniaturized incubator (101).

8. Platform (100) according to claim 1, **characterized in that** the humidification system comprises: a bowl (106) able to contain water; and heating elements (111), able to heat said water, fixed inside the bowl (106).

9. Platform (100) according to claims 6 and 7, **characterized in that** the miniaturized incubator (101) comprises, fixed in correspondence with a lower portion of the upper panel (101f), a film made of FEP, Fluorinated Ethylene Propylene, configured to close the miniaturized incubator (101) when the first covering element (104) or the second covering element (105) are not inserted inside the upper panel (101f), and configured for the passage of the UV-C radiation generated by the sterilization system when the second covering element (105) is inserted inside said upper panel (101f).

10. Platform (100) according to claim 3, **characterized in that** the heating system, the sterilization system and the humidification system are connected to the electronic unit (116) by means of electrical cables passing through openings made on surfaces of the left side panel (101c) and further openings made on corresponding surfaces of the case (117) of the control module (115), and **in that** portions of sheaths covering the plurality of sensors are housed inside the miniaturized incubator (101), and remaining portions of said sheaths come out of the miniaturized incubator (101) by means of the openings made on the surfaces of the left side panel (101c) and further openings made on surfaces of the right side panel (101d).

11. Platform (100) according to claim 1, **characterized in that** the electronic unit (116) is configured to run a PID, Proportional Integral Derivative, control logic for a real-time management of the heating system, of the sterilization system and of the humidification system of the miniaturized incubator (101).

12. Platform (100) according to claims 6 and 8, **characterized in that** the plurality of sensors includes:
- a pH sensor (108);
- a DO, Dissolved Oxygen, sensor (109);
- a temperature and humidity sensor (114), configured to detect the humidity and temperature of the microenvironment of the cell culture inside the miniaturized incubator (101);
- a level sensor (107) configured to detect the level of the water contained in the bowl (106);
- a temperature sensor (120) configured to detect the temperature of the heating glass (104a);
- a thermocouple, connected to the heating elements (111) able to heat the water contained in the bowl (106), able to detect the temperature of said water.

13. Platform (100) according to claims 8 and 11, **characterized in** comprising a micro-pump, connected to a tank and to the electronic unit (116), positioned externally to the miniaturized incubator (101) and configured to introduce water into the bowl (106), according to an automated filling managed by means of the PID control logic running on said electronic unit (116).

14. Platform (100) according to claim 1, **characterized in** comprising a solenoid valve, connected to the electronic unit (116), configured for the introduction through pipes (121) of a 5% CO₂ mixture into the microenvironment of the cell culture, said CO₂ mixture being able to control the pH of the cell culture.

15. Platform (100) according to claim 3, **characterized in that** the lower panel (101a), the front panel (101b), the left side panel (101c), the right side panel (101d), the rear panel (101e), the upper panel (101f) and the base stage (102) are made of PMMA by means of micro-milling and 3D printing.

16. Platform (100) according to claim 1, **characterized in that** the at least one microscope or spectroscope (300) is configured for a real-time observation of the static and dynamic cell culture supports through the optical window (112).

17. Platform (100) according to claim **1, characterized in** comprising a wireless data transmission unit connected to the electronic unit (116), and at least one cloud storage module able to save data processed by the electronic unit (116) and transmitted to said at least one cloud storage module by means of the wireless data transmission unit.

18. Platform (100) according to claim **3, characterized in that** the body has a plan section having a length comprised between 143 mm and 223 mm and a width comprised between 80 mm and 160 mm, said body having a height comprised between 22 mm and 62 **mm,** and **in that** the base stage (102) has a substantially rectangular shape having a length comprised between 205 mm and 265 **mm,** and a width comprised between 124 mm and 184 mm.

## Patentansprüche

1. Plattform (100) zum Screening statischer und dynamischer Zellkulturträger, umfassend:
- einen miniaturisierten Inkubator (101) für Zellkulturen, bestehend aus: einem Körper umfassend eine Frontplatte (101b), mehrere im Körper untergebrachte Sensoren sowie ein Heizsystem, ein Sterilisationssystem und ein Befeuchtungssystem, die so konfiguriert sind, dass sie im Inneren des miniaturisierten Inkubators (101) vordefinierte und optimale Bedingungen, typisch von einer Zellkultur-Mikroumgebung, reproduzieren,
wobei der miniaturisierte Inkubator (101) statische und dynamische Zellkulturträger abnehmbar lagert ;
- ein Steuermodul (115), bestehend aus: einem Gehäuse (117), an den Körper des miniaturisierten Inkubators (101) angrenzend und einer in dem Gehäuse (117) untergebrachten Elektronikeinheit (116), die mit den mehreren Sensoren, dem Heizsystem, dem Sterilisationssystem und dem Befeuchtungssystem verbunden ist ;
- eine Basisplatte (102), die abnehmbar unterhalb des miniaturisierten Inkubators (101) befestigt ist und mindestens ein Schnittstellenelement in Form eines Adapters oder eines Adaptersystems aufweist, die die Plattform (100) abnehmbar mit mindestens einem Mikroskop oder Spektroskop (300) verbinden;
**dadurch gekennzeichnet, dass** die Frontplatte (101b) Gleitmittel (113) und Führungen (103) für die Bewegung der Gleitmittel (113) aufweist, wobei die Gleitmittel (113) aus einem Schlitten bestehen, der abwechselnd die statischen und dynamischen Zellkulturträger trägt und in den miniaturisierten Inkubator (101) einführt, und mit einem optischen Fenster (112) versehen sind, das über einer Außenfläche des Schlittens (113) positioniert ist, wobei in korrespondierenden Position mit dem optischen Fenster (112) außerhalb des miniaturisierten Inkubators (101) mindestens ein Mikroskop oder ein Spektroskop (300) abnehmbar positioniert sind.

2. Plattform (100) nach Anspruch 1, **dadurch gekennzeichnet, dass** die statischen Zellkulturträger aus Petrischalen, Multiwellplatten oder Objektträgern bestehen, und dass die dynamischen Zellkulturträger aus mikrofluidischen Vorrichtungen (200) bestehen.

3. Plattform (100) nach Anspruch 1, **dadurch gekennzeichnet, dass** der Körper eine im Wesentlichen quaderförmige Gestalt aufweist und aus einer unteren Platte (101a), einer linken Seitenplatte (101c), einer rechten Seitenplatte (101d), einer hinteren Platte (101e), einer oberen Platte (101f) und der Vorderplatte (101b) besteht.

4. Plattform (100) nach Anspruch 3, **dadurch gekennzeichnet, dass** die Basisplatte (102) abnehmbar unterhalb des miniaturisierten Inkubators (101) in korrespondierender Position mit der unteren Platte (101a) befestigt ist.

5. Plattform (100) nach Anspruch 3, **dadurch gekennzeichnet, dass** die obere Platte (101f) hohl ist und zusätzliche Führungen für die abnehmbare Einsetzung von Abdeckelementen aufweist, die so konfiguriert sind, dass sie den Körper des miniaturisierten Inkubators (101) bedecken.

6. Plattform (100) nach Anspruch 5, **dadurch gekennzeichnet, dass** das Heizsystem ein erstes Abdeckelement (104) des Körpers des miniaturisierten Inkubators (101) umfasst, das in den zusätzlichen Führungen der oberen Platte (101f) gleiten kann und mit einem transparenten und elektrisch leitfähigen Heizglas (104a) versehen ist, wobei das Heizglas (104a) in der Lage ist, die Temperatur der Zellkultur-Mikroumgebung im Inneren des miniaturisierten Inkubators (101) zu regulieren.

7. Plattform (100) nach Anspruch 5, **dadurch gekennzeichnet, dass** das Sterilisationssystem ein zweites Abdeckelement (105) des Körpers des miniaturisierten Inkubators (101) umfasst, das in der Lage ist, in den zusätzlichen Führungen der mit mehreren LED (105a) versehenen oberen Platte (101f) zu gleiten, wobei die mehreren LED (105a) so konfiguriert sind, dass sie eine UV-C-Strahlung erzeugen, die zur Sterilisation der Mikroumgebung der Zellkultur im Inneren des miniaturisierten Inkubators (101) geeignet ist.

8. Plattform (100) nach Anspruch 1, **dadurch gekennzeichnet, dass** das Befeuchtungssystem eine Schale (106) zur Aufnahme von Wasser und Heizelemente (111) zum Erhitzen des Wassers umfasst, die in der Schale (106) befestigt sind.

9. Plattform (100) nach Anspruch 6 und 7, **dadurch gekennzeichnet, dass** der miniaturisierte Inkubator (101) gegenüber einem unteren Abschnitt der oberen Platte (101f) eine FEP (Fluorethylen-Propylen) - Folie aufweist, die den miniaturisierten Inkubator (101) verschließt, wenn das erste Bedeckungselement (104) oder das zweite Bedeckungselement (105) nicht in die obere Platte (101f) eingesetzt ist, und die den Durchtritt der vom Sterilisationssystem erzeugten UV-C-Strahlung ermöglicht, wenn das zweite Bedeckungselement (105) in die obere Platte (101f) eingesetzt ist.

10. Plattform (100) nach Anspruch 3, **dadurch gekennzeichnet, dass** das Heizsystem, das Sterilisationssystem und das Befeuchtungssystem über elektrische Kabel, die durch Öffnungen in der linken Seitenwand (101c) und zusätzliche Öffnungen in entsprechenden Oberflächen des Gehäuses (117) des Steuermoduls (115) verlaufen, mit der elektronischen Einheit (116) verbunden sind, und dadurch, dass Teile der die Sensoren bedeckenden Hüllen im Inneren des miniaturisierten Inkubators (101) untergebracht sind und die restlichen Teile dieser Hüllen durch Öffnungen in der linken Seitenwand (101c) und zusätzliche Öffnungen in der rechten Seitenwand (101d) aus dem miniaturisierten Inkubator (101) herausragen.

11. Plattform (100) nach Anspruch 1, **dadurch gekennzeichnet, dass** die elektronische Einheit (116) zur Ausführung einer PID - Proportional-Integral-Differential Kontrolllogik für ein Echtzeitmanagement der Heizsystems, des Sterilisationssystems und des Befeuchtungssysteme des miniaturisierten Inkubators (101), konfiguriert ist.

12. Plattform (100) nach Anspruch 6 und 8, **dadurch gekennzeichnet, dass** die Mehrzahl der Sensoren Folgendes umfasst:
- einen pH-Sensor (108);
- einen DO, Dissolved Oxygen, Sensor (109);
- einen Temperatur- und Feuchtigkeitssensor (114), konfiguriert zur Detektion der Feuchtigkeit und der Temperatur der Zellkultur-Mikroumgebung innerhalb des miniaturisierten Inkubators (101);
- einen Füllstand-Sensor (107) konfiguriert zur Detektion des Wasserstands in der Schale (106);
- ein Temperatursensor (120) konfiguriert zur Detektion der Temperatur des Heizglases (104a)
- ein Thermoelement, das mit den Heizelementen (111) verbunden und in de Lage ist, das in der Schale (106) enthaltene Wasser zu heizen und die Temperatur dieses Wassers zu detektieren.

13. Plattform (100) nach Anspruch 8 und 11, **dadurch gekennzeichnet, dass** sie eine Mikropumpe umfasst, die mit einem Tank und mit der elektronischen Einheit (116) verbunden, außerhalb des miniaturisierten Inkubators (101) angeordnet und so konfiguriert ist, dass sie Wasser gemäß einem automatisierten Füllvorgang, der von der auf der elektronischen Einheit (116) laufenden PID-Kontrolllogik gesteuert wird, in den Behälter (106) einleitet.

14. Plattform (100) nach Anspruch 1, **dadurch gekennzeichnet, dass** sie ein mit der Elektronikeinheit (116) verbundenes Magnetventil umfasst, das so konfiguriert ist, dass es über Leitungen (121) ein 5%iges CO₂-Gemisch in die Zellkultur-Mikroumgebung einleitet, wobei das CO₂-Gemisch den pH-Wert der Zellkultur regulieren kann.

15. Plattform (100) nach Anspruch 3, **dadurch gekennzeichnet, dass** die untere Platte (101a), die vordere Platte (101b), die linke Seitenplatte (101c), die rechte Seitenplatte (101d), die hintere Platte (101e), die obere Platte (101f) und die Basisplatte (102) aus PMMA mittels Mikrofräsen und 3D-Druck gefertigt sind.

16. Plattform (100) nach Anspruch 1, **dadurch gekennzeichnet, dass** mindestens ein Mikroskop oder ein Spektroskop (300) zur Echtzeitbeobachtung statischer und dynamischer Zellkulturträger durch das optische Fenster (112) konfiguriert sind.

17. Plattform (100) nach Anspruch 1, **dadurch gekennzeichnet, dass** sie eine mit der elektronischen Einheit (116) verbundene drahtlose Datenübertragungseinheit und mindestens ein Cloud-Speichermodul für die von der elektronischen Einheit (116) verarbeiteten und mittels der drahtlosen Datenübertragungseinheit an dieses Cloud-Speichermodul übertragenen Daten umfasst.

18. Plattform (100) nach Anspruch 3, **dadurch gekennzeichnet, dass** der Körper einen flachen Abschnitt mit einer Länge zwischen 143 mm und 223 mm und einer Breite zwischen 80 mm und 160 mm aufweist, wobei der Körper eine Höhe zwischen 22 mm und 62 mm besitzt, und dass die Basisplatte (102) eine im Wesentlichen rechteckige Form mit einer Länge zwischen 205 mm und 265 mm und mit einer Breite zwischen 124 mm und 184 mm aufweist.

## Revendications

1. Plateforme (100) de criblage de supports de culture cellulaire statiques et dynamiques, comprenant :
- un incubateur miniaturisé (101) pour cultures cellulaires comprenant : un corps comprenant un panneau avant (101b), une pluralité de capteurs logés dans le corps ; et un système de chauffage, un système de stérilisation et un système d'humidification, configurés pour reproduire, à l'intérieur dudit incubateur miniaturisé (101), des conditions prédéfinies et optimales, propres à un microenvironnement d'une culture cellulaire,
ledit incubateur miniaturisé (101) logeant de manière amovible des supports de culture cellulaire statiques et dynamiques ;
- un module de commande (115) comprenant : un boitier (117) adjacent au corps de l'incubateur miniaturisé (101) et une unité électronique (116) logée dans ledit boitier (117) et connectée à la pluralité de capteurs, au système de chauffage, au système de stérilisation et au système d'humidification ;
- une étage de base (102), fixée de manière amovible au-dessous de l'incubateur miniaturisé (101), comprenant au moins un moyen d'interface constitué d'un adaptateur ou d'un système d'adaptateurs connectant de manière amovible ladite plateforme (100) à au moins un microscope ou spectroscope (300) ;
**caractérisée en ce que** le panneau avant (101b) comprend un moyens de glissement (113) et des guides (103) pour le mouvement des moyens de glissement (113), lesdits moyens de glissement (113) étant constitués d'un chariot, soutenant et introduisant de manière amovible, à l'intérieur de l'incubateur miniaturisé (101), alternativement lesdits supports de culture cellulaire statiques et dynamiques, pourvus d'une fenêtre optique (112) positionnée au-dessus d'une surface extérieure dudit chariot (113), en correspondance avec ladite fenêtre optique (112) étant positionné de manière amovible à l'extérieur de l'incubateur miniaturisé (101), au moins un microscope ou un spectroscope (300).

2. Plateforme (100) selon la revendication 1, **caractérisée en ce que** les supports de culture cellulaire statiques sont constitués de boîtes de Petri, de plaques multipuits ou de lames, et **en ce que** les supports de culture cellulaire dynamiques sont constitués de dispositifs micro-fluidiques (200).

3. Plateforme (100) selon la revendication 1, **caractérisée en ce que** le corps a une forme sensiblement parallélépipédique et est constitué d'un panneau inférieur (101a), d'un panneau latéral gauche (101c), d'un panneau latéral droit (101d), d'un panneau arrière (101e), d'un panneau supérieur (101f) et du panneau avant (101b).

4. Plateforme (100) selon la revendication 3, **caractérisée en ce que** l'étage de base (102) est fixée de manière amovible au-dessous de l'incubateur miniaturisé (101) en correspondance avec le panneau inférieur (101a).

5. Plateforme (100) selon la revendication 3, **caractérisée en ce que** le panneau supérieur (101f) est creux et comporte des guides supplémentaires pour une insertion amovible d'éléments de revêtement configurés pour le revêtement du corps de l'incubateur miniaturisé (101).

6. Plateforme (100) selon la revendication 5, **caractérisée en ce que** le système de chauffage comprend un premier élément de revêtement (104) du corps de l'incubateur miniaturisé (101), pouvant coulisser dans les guides supplémentaires du panneau supérieur (101f), pourvu d'un verre chauffant transparent et électriquement conducteur (104a), ledit verre chauffant (104a) pouvant régler la température du microenvironnement de la culture cellulaire à l'intérieur de l'incubateur miniaturisé (101).

7. Plateforme (100) selon la revendication 5, **caractérisée en ce que** le système de stérilisation comprend un deuxième élément de revêtement (105) du corps de l'incubateur miniaturisé (101), pouvant coulisser dans les guides supplémentaires du panneau supérieur (101f), pourvu d'une pluralité de LED (105a), ladite pluralité de LED (105A) étant configurés pour générer un rayonnement UV-C adapté à stériliser le microenvironnement de la culture cellulaire à l'intérieur de l'incubateur miniaturisé (101).

8. Plateforme (100) selon la revendication 1, **caractérisée en ce que** le système d'humidification comprend un bac (106) pouvant contenir de l'eau, et des éléments de chauffage (111) pouvant chauffer ladite eau, fixés à l'intérieur dudit bac (106).

9. Plateforme (100) selon les revendications 6 et 7, **caractérisée en ce que** l'incubateur miniaturisé (101) comprend, fixé en correspondance avec une partie inférieure du panneau supérieur (101f), un film en FEP, fluoréthylène-propylène), configuré pour fermer l'incubateur miniaturisé (101), lorsque le premier élément de revêtement (104) ou le deuxième élément de revêtement (105) ne sont pas insérés à l'intérieur du panneau supérieur (101f), et configuré pour le passage du rayonnement UV-C généré par le système de stérilisation lorsque le deuxième élément de revêtement (105) est inséré à l'intérieur dudit panneau supérieur (101f).

10. Plateforme (100) selon la revendication 3, **caractérisée en ce que** le système de chauffage, le système de stérilisation et le système d'humidification sont connectés à l'unité électronique (116) au moyen de câbles électriques passant par des ouvertures pratiquées sur des surfaces du panneau latéral gauche (101c) et d'ouvertures supplémentaires pratiquées sur des surfaces correspondantes du boitier (117) du module de commande (115), et **en ce que** des parties de gaines recouvrant la pluralité de capteurs sont logées à l'intérieur de l'incubateur miniaturisé (101), et que des portions restantes desdites gaines émergent de l'incubateur miniaturisé (101) au moyen des ouvertures pratiquées dans les surfaces du panneau latéral gauche (101c) et d'ouvertures supplémentaires pratiquées dans les surfaces du panneau latéral droit (101d).

11. Plateforme (100) selon la revendication 1, **caractérisée en ce que** l'unité électronique (116) est configurée pour exécuter une logique de commande PID, Proportionnel Intégral Dérivé, pour une gestion en temps réel du système de chauffage, du système de stérilisation et du système d'humidification de l'incubateur miniaturisé (101).

12. Plateforme (100) selon les revendications 6 et 8, **caractérisée en ce que** la pluralité des capteurs comprend :
- un capteur de pH (108) ;
- un capteur DO, Oxygène Dissous (109) ;
- un capteur de température et d'humidité (114), configuré pour la détection de l'humidité et de la température du microenvironnement de la culture cellulaire à l'intérieur de l'incubateur miniaturisé (101) ;
- un capteur de niveau (107) configuré pour la détection du niveau de l'eau contenue dans le bac (106) ;
- un capteur de température (120) configuré pour la détection de la température du verre chauffant (104a) ;
- un thermocouple, connecté aux éléments chauffants (111) pouvant chauffer l'eau contenue dans le bac (106), capable de détecter la température de ladite eau.

13. Plateforme (100) selon les revendications 8 et 11, **caractérisée en ce qu'**elle comprend une micro-pompe, connectée à un réservoir et à l'unité électronique (116), positionnée à l'extérieur de l'incubateur miniaturisé (101) et configurée pour introduire de l'eau dans le bac (106), selon un remplissage automatisé géré par la logique de commande PID exécutée sur ladite unité électronique (116).

14. Plateforme (100) selon la revendication 1, **caractérisée en ce qu'**elle comprend une électrovanne, connectée à l'unité électronique (116), configurée pour l'introduction, par des conduites (121), d'un mélange à 5 % de CO₂ dans le microenvironnement de la culture cellulaire, ledit mélange de CO₂ étant capable de régler le pH de la culture cellulaire.

15. Plateforme (100) selon la revendication 3, **caractérisée en ce que** le panneau inférieur (101a), le panneau avant (101b), le panneau latéral gauche (101c), le panneau latéral droit (101d), le panneau arrière (101e), le panneau supérieur (101f) et l'étage de base (102) sont réalisés en PMMA par micro-fraisage et par impression 3D.

16. Plateforme (100) selon la revendication 1, **caractérisée en ce qu'**au moins un microscope ou un spectroscope (300) est configuré pour une observation en temps réel des supports de culture cellulaire statiques et dynamiques à travers la fenêtre optique (112).

17. Plateforme (100) selon la revendication 1, **caractérisée en ce qu'**elle comprend une unité de transmission de données sans fil connectée à l'unité électronique (116), et au moins un module de stockage cloud pouvant sauver des données traitées par l'unité électronique (116) et transmises audit au moins un module de stockage cloud au moyen de l'unité de transmission de données sans fil.

18. Plateforme (100) selon la revendication 3, **caractérisée en ce que** le corps présente une section plane avec une longueur comprise entre 143 mm et 223 mm et une largeur comprise entre 80 mm et 160 mm, ledit corps ayant une hauteur comprise entre 22 mm et 62 mm, et **en ce que** l'étage de base (102) a une forme sensiblement rectangulaire avec une longueur comprise entre 205 mm et 265 mm et une largeur comprise entre 124 mm et 184 mm.
